# EUROPEAN PATENT SPECIFICATION

(11) **EP 2 869 830 B1**
(45) Date of publication and mention of the grant of the patent: **12.10.2016**
(21) Application number: 13734772.0
(22) Date of filing: 08.07.2013
(51) Int. Cl.: A61K 38/28, A61P 3/10, C07K 14/62

(54) **NOVEL USE OF INSULIN DERIVATIVES**
NEUARTIGE VERWENDUNG VON INSULINDERIVATEN
NOUVELLE UTILISATION DE DÉRIVÉS DE L'INSULINE

(30) Priority: 09.07.2012 EP 12175525; 16.07.2012 US 201261671912 P; 05.03.2013 EP 13157728; 13.03.2013 US 201361778556 P
(43) Date of publication of application: 13.05.2015
(73) Proprietor: Novo Nordisk A/S, 2880 Bagsværd (DK)
(72) Inventor: PRIDAL, Lone, DK-2880 Bagsværd (DK); NAVER, Helle, DK-2880 Bagsværd (DK); MADSEN, Peter, DK-2880 Bagsværd (DK); KJELDSEN, Thomas Børglum, DK-2880 Bagsværd (DK)
(86) International application number: PCT/EP2013/064392
(87) International publication number: WO 2014/009316

(56) References cited:
- WO-A1-2009/115469
- WO-A1-2010/049488
- WO-A1-2011/161125

## Description

### FIELD OF THIS INVENTION

This invention relates to insulin derivatives having an extremely long duration of action. Said derivatives can be used to treat diabetes and aspects related thereto. In a preferred aspect, these derivatives are only administered around once weekly to the patients.

### BACKGROUND OF THIS INVENTION

Insulin is a polypeptide hormone secreted by β-cells of the pancreas. Insulin consists of two polypeptide chains designated the A and B chains which are linked together by two inter-chain disulphide bridges. In human, porcine and bovine insulin, the A and B chains contains 21 and 30 amino acid residues, respectively. However, from species to species, there are variations among the amino acid residues present in the different positions in the two chains. The widespread use of genetic engineering has made it possible to prepare analogues of natural occurring insulins by exchanging, deleting and adding one of more of the amino acid residues.

Insulin is used for the treatment of diabetes and diseases connected therewith or resulting from it. Insulin is essential in maintaining normal metabolic regulation. Usually, insulin is administered by injections (subcutaneously). Unfortunately, many diabetics are unwilling to undertake intensive therapy due to the discomfort associated with the many injections required to maintain close control of blood glucose levels. In the last decades, it has turned out that it is extremely important for a diabetic patient to maintain close control of the blood glucose level.

For decades, insulin preparations with different duration of action have been developed and put on the market and general examples of such preparations are long-acting insulin preparations, medium acting insulin preparations and fast acting insulin preparations. Many patients take 2-4 injections per day. For example, before a meal, many patients take a fast acting insulin preparation which shall control the glucose level after the meal, such treatments being designated prandial treatment. However, in order to control the glucose level at other moments than close to the moments of eating, diabetics have to take an insulin preparation with a longer duration of action, e.g., before they go to sleep, and this can be designated basal treatment. Hence, many diabetics take one injection every evening before they go to sleep. Even though such insulin preparations are effective in controlling the glucose level for about one night and day, they are not sufficient for controlling the glucose level for more than one night and day. No basal insulin products are approved for other than daily subcutaneous injection. The discomfort of a large number of daily injections can, for example, be diminished by using insulin derivatives having an extremely long duration of action.

Claim 1 in WO 2010/049488 mentions "administering ... effective dosages of the insulin derivative ... said dosages are administered at intervals longer than 24 hours". According to the abstract in J.Controlled Release 104 (2005), 447-60, "When doses [of PEGylated human insulin encapsulated in PLGA] were given [to animals] at 7-day intervals, steady state drug levels were achieved after only 2 doses". Claim 25 in WO 98/05361 mentions "A method wherein ... effective doses of N-(Fmoc)₂-insulin and N-(Fmoc)₃-insulin are administered to the patient at 5-8 day intervals". In example 107 of WO 2011/161125, it is mentioned that acylated insulin derivatives of the invention (insulin derivatives having two or more cysteine substitutions to allow for one or more additional disulphide bonds) are administered to rats, plasma is collected at time points 0-96 hours after dosing and the plasma is assayed for glucose. WO 2009/115469 relates to acylated insulin analogues that are able to give a satisfactory control of the blood glucose level following once daily administration, see page 2 line 15.

It is very desirable for the diabetics, if insulin preparations which are to be administered only say about once weekly and which would give a satisfactory basal treatment were available.

### OBJECTS OF THIS INVENTION

The object of this invention is to overcome or ameliorate at least one of the disadvantages of the prior art, or to provide a useful alternative.

Another aspect of this invention relates to the furnishing of insulin derivatives which have a so long time of action that it is sufficient to administer them with a frequency of about once weekly in order for the diabetic patient to get a sufficient basal administration of insulin.

Another aspect of this invention relates to the furnishing of insulin derivatives which have a long action profile.

Another aspect of this invention relates to the furnishing of insulin derivatives which increases convenience for the patients.

Another aspect of this invention relates to the furnishing of insulin derivatives which have a constant time-action profile for a period of time of about 1 week.

Another aspect of this invention relates to the furnishing of insulin derivatives which have a flat action profile for a period of time of about 1 week.

Another aspect of this invention relates to the furnishing of insulin derivatives which have increased solubility.

Another aspect of this invention relates to the furnishing of insulin derivatives which have increased solubility in presence of zinc ions.

Another aspect of this invention relates to the furnishing of insulin derivatives which have increased solubility in presence of zinc ions and salt, such as sodium chloride.

### DEFINITIONS

As defined herein, the term "diabetes" or "diabetes mellitus" includes type 1 diabetes, type 2 diabetes, gestational diabetes (during pregnancy) and other states that cause hyperglycaemia. The term is used for a metabolic disorder in which the pancreas produces insufficient amounts of insulin, or in which the cells of the body fail to respond appropriately to insulin thus preventing cells from absorbing glucose. As a result, glucose builds up in the blood.

Type 1 diabetes, also called insulin-dependent diabetes mellitus (IDDM) and juvenile-onset diabetes, is caused by B-cell destruction, usually leading to absolute insulin deficiency. Type 2 diabetes, also known as non-insulin-dependent diabetes mellitus (NIDDM) and adult-onset diabetes, is associated with predominant insulin resistance and thus relative insulin deficiency and/or a predominantly insulin secretory defect with insulin resistance.

As defined herein, the, the term "formulation" is used synonymously with the term "composition".

As defined herein, the, the following abbreviations are used: "gGlu" or "γGlu" for gamma L-glutamyl and "OEG" for the amino acid with the formula NH₂-(CH₂)₂-O-(CH₂)₂-O-CH₂-COOH corresponding to the group or residue -NH-(CH₂)₂-O-(CH₂)₂-O-CH₂-CO- also designated [2-(2-aminoethoxy)ethoxy]methylcarbonyl.

### DETAILED DESCRIPTION OF THIS INVENTION

Human insulin consists of two polypeptide chains, interconnected by two cystine disulphide bridges. The primary amino acid sequence for the human insulin A chain (21 amino acids) is n-G I V E Q C C T S I C S L Y Q L E N Y C N-c (SEQ ID NO: 1). The primary amino acid sequence for the human insulin B chain (30 amino acids) is n-F V N O H L C G D H L V E A L Y L V C G E R G F F Y T P K T-c (SEQ ID NO: 2).

It has, surprisingly, been found that the insulin derivatives recited in claim 1 have an extremely long duration of action. These insulin derivatives have a so retarded duration of action that, to many patients, it can be used as the only insulin product for basal administration and that it is sufficient to administer it only about once weekly. A specific example of these insulin derivatives is A14E, B16H, B25H, B29K(N^{ε}-eicosanedioyl-γGlu-[2-(2-{2-[2-(2-aminoethoxy)ethoxy]acetylaminolethoxy)ethoxy]acetyl), desB30 human insulin (herein designated Compound 1). This compound can also be designated A14E, B16H, B25H, B29K(N^{ε}-eicosandioyl-γGlu-OEG-OEG), desB30 human insulin, or alternatively A14E, B16H, B25H, B29K(N^{ε}-eicosandioyl-γGlu-2xOEG), desB30 human insulin, and has the following formula:

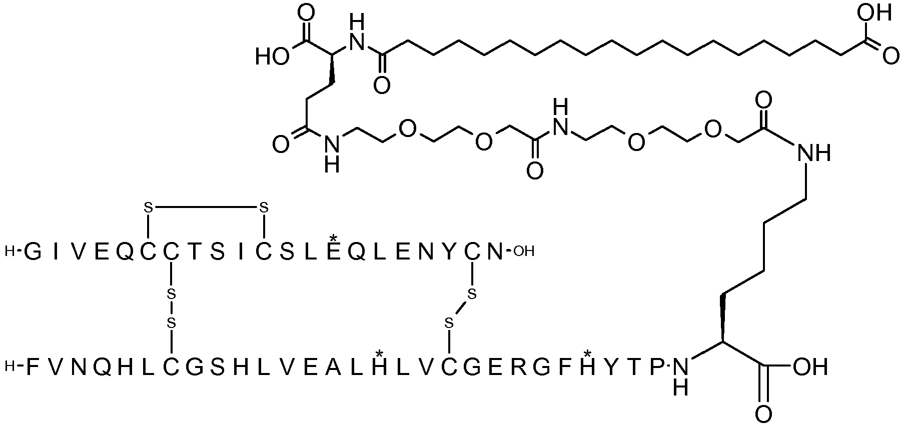

The above compound is mentioned in example 33 in WO 2009/115469.

Examples of other specific insulin derivatives of for use according to the invention are A14E, B16H, B25H, B29K(N^{ε}-hexadecandioyl-γGlu), desB30 human insulin (herein designated Compound 2); A14E, B16H, B25H, B29K(N^{ε}-eicosanedioyl-γGlu), desB30 human insulin (herein designated Compound 3); A14E, B25H, desB27, B29K(N^{ε}-(octadecandioyl-γGlu), desB30 human insulin (herein designated Compound 4); A14E, B25H, desB27, B29K(N^{ε}-octadecanedioyl-γGlu-OEG-OEG), desB30 human insulin (herein designated Compound 5); and A14E, B25H, B29K(N^{ε}-octadecandioyl-γGlu-OEG-OEG), desB30 human insulin (herein designated Compound 6). The formulae of the five last-mentioned compounds are stated in examples 27, 60, 151, 57 and 9, respectively, in WO 2009/115469.

Biophysics studies show that Compound 1 has different behaviour than ordinary insulin molecules and, similarly, this is expected to apply for the other insulin derivatives recited in the claims.

The *in vivo* potency of Compound 1 in pigs and dogs was estimated to be approximately 200% relative to insulin glargine.

*In vivo* pharmacology of Compound 1 in pigs has shown that dosing of a 4.2 mM solution, the absolute bioavailability is 90% relative to intravenously administration of the same compound.

Pharmaceutical formulations containing an insulin derivative for use according to the invention can be prepared in a manner known *per se,* i.e. by using the additives usually used in similar insulin formulations.

The insulin formulations are administered to the patients in a manner known perse, e.g. according to the general knowledge of the patient combined with the general knowledge of the physician. This invention is best used at the convenience of the patient. Therefore, specific administration intervals will be explored for each patient where dosages are administered less than daily. The final mode of use thus depends both on the product's capabilities and on the disposition and preference of the patient. This is due to the fact that the effect of any insulin product depends on the insulin need of the individual patient and the sensitivity to the pharmacodynamic actions of said insulin and lastly also to the preferences of the patient in a given situation. These conditions may change over time, both in terms of longer periods (years) and from day to day. The optimal dose level for any patient will depend on a variety of factors including the age, body weight, physical activity, and diet of the patient, on a possible combination with other drugs, and on the severity of the state to be treated. It is recommended that the dosage regimen be determined for each individual patient by those skilled in the art in a similar way as for known insulin formulations, however taking into consideration the present teachings concerning dosage intervals.

For the convenience of the patients, it is presumed that they prefer that the time interval (time lag) from the administration of an insulin derivative for use according to the invention to the next administration of this derivative has the same length, or approximately the same length, counted in number of days. It can even be expected that the patients will prefer that the administration of an insulin derivative for use according to the invention takes place only once weekly, i.e. on the same day in the week, e.g. on every sunday. This will be an administration of an insulin derivative for use according to the invention every 7^{th} day and not more frequently on an average calculated for a period of time of 1 month, 6 months or 1 year. For some patients, it may be desirable to administer the insulin derivative every 6^{th} day or approximately every 6^{th} day and not more frequently on an average calculated for a period of time of 1 month, 6 months or 1 year. For other patients, it may be desirable to administer an insulin derivative for use according to the invention every 5^{th} day or approximately every 5^{th} day and not more frequently on an average calculated for a period of time of 1 month, 6 months or 1 year. For other patients, it may be desirable to administer the derivative every 4^{tn} day or approximately every 4^{th} day and not more frequently on an average calculated for a period of time of 1 month, 6 months or 1 year. Even other patients may find it advantageous to administer an insulin derivative for use according to the invention only twice weekly, e.g., with an interval of about 3-4 days between each administration and not more frequently on an average calculated for a period of time of 1 month, 6 months or 1 year. For some patients, it may be desirable to administer the derivative every 3^{rd} day or approximately every 3^{rd} day and not more frequently on an average calculated for a period of time of 1 month, 6 months or 1 year. For other patients, it may be desirable to administer an insulin derivative for use according to the invention every 2^{nd} day or approximately every 2^{nd} day and not more frequently on an average calculated for a period of time of 1 month, 6 months or 1 year. For some patients, it may be desirable to administer the derivative every 8^{th} day or approximately every 8^{th} day and not more frequently on an average calculated for a period of time of 1 month, 6 months or 1 year. Even other patients may not administer an insulin derivative for use according to the invention with a time interval of precisely the same length (counted in days), week after week, month after month or year after year. Some patients may administer an insulin derivative for use according to the invention sometime in the time interval from every 6^{th} to every 8^{th} day and not more frequently on an average calculated for a period of time of 1 month, 6 months or 1 year. Other patients may administer an insulin derivative for use according to the invention sometime in the time interval from every 5^{th} to every 7^{th} day and not more frequently on an average calculated for a period of time of 1 month, 6 months or 1 year. Even other patients may administer an insulin derivative for use according to the invention sometime in the time interval from every 4^{th} to every 8^{th} day and not more frequently on an average calculated for a period of time of 1 month, 6 months or 1 year.

The time intervals mentioned here are to be understood as average time intervals within a period of time of say weeks, months or years. Here, it is intended that the term "day" covers 24 hours (i.e. a day and night) and, for the sake of easiness, a number of hours which is not divisible by 24 is to be rounded up to a whole number of days. Hence, e.g., 30 hours corresponds to 1 day and 40 hours corresponds to 2 days. Obviously, the above intervals of administration are to be calculated for each person.

The patients may have a daily basal insulin requirement of above about 0.2 IU/kg/day and below about 1 IU/kg/day and, furthermore, the patients may have a total (i.e. basal plus prandial) daily insulin requirement of above about 1 IU/kg/day. However, these ranges may vary considerably from patient to patient and may for several patients be somewhat outside the ranges mentioned here.

Diseases and conditions which are the primary targets for this invention are diabetes mellitus (type 1 or 2) or other conditions characterized by hyperglycaemia, but also metabolic diseases and conditions in general where the metabolic effects of insulin has a clinical relevance or are of interest, such as pre-diabetes, impaired glucose tolerance, metabolic syndrome, obesity, cachexia, *in vivo* beta-cell loss/death, excessive appetite, and inflammation. All these types of conditions are known to or believed to benefit from a stable metabolic state in the subject who has the disease or condition. At any rate, any therapeutic regimen where administration of insulin is included may be modified by implementing the current teachings, meaning that such therapies will include administration of prolonged-profile-of-action insulins according to the teachings provided herein.

In order to exercise this invention, an insulin preparation may be administered parenterally to patients in need of such a treatment. Parenteral administration may be performed by subcutaneous, intramuscular or intravenous injection by means of a syringe, optionally a pen-like syringe. Alternatively, parenteral administration can be performed by means of an infusion pump. Further options are to administer the insulin composition nasally or pulmonary, preferably in compositions, powders or liquids, specifically designed for the purpose.

Embodiments of this invention include those wherein administration of an insulin derivative for use according to the invention is supplemented with more frequent administrations of a fast-acting naturally occurring insulin, insulin analogue or insulin derivative and/or administration of a non-insulin anti-diabetic drug. In one embodiment of this invention, administration of an insulin derivative for use according to the invention is supplemented with administration of a non-insulin anti-diabetic drug, such as metformin.

### PREFERRED FEATURES OF THIS INVENTION

1. An insulin derivative for use in the treatment of diabetes, which derivative is A14E, B16H, B25H, B29K(N^{ε}-eicosanedioyl-gGlu-[2-(2-{2-[2-(2-aminoethoxy)ethoxy]acetylamino}ethoxy)ethoxy]acetyl), desB30 human insulin (Compound 1).
2. An insulin derivative for use in the treatment of diabetes, which derivative is A14E, B16H, B25H, B29K(N^{ε}-hexadecandioyl-γGlu), desB30 human insulin (Compound 2).
3. An insulin derivative for use in the treatment of diabetes, which derivative is A14E, B16H, B25H, B29K(N^{ε}-eicosanedioyl-γGlu), desB30 human insulin (Compound 3).
4. An insulin derivative for use in the treatment of diabetes, which derivative is A14E, B25H, desB27, B29K(N^{ε}-octadecandioyl-γGlu), desB30 human insulin (Compound 4).
5. An insulin derivative for use in the treatment of diabetes, which derivative is A14E, B25H, desB27, B29K(*N^{ε}*-octadecanedioyl-γGlu-OEG-OEG), desB30 human insulin (Compound 5).
6. An insulin derivative for use in the treatment of diabetes, which derivative is A14E, B25H, B29K(*N^{ε}*-octadecandioyl-γGlu-OEG-OEG), desB30 human insulin (Compound 6).
7. The insulin derivative for use according to the invention, wherein the currently or repeatedly treatment for diabetes with a compound of formula I lasts for more than 1 month.
8. The insulin derivative for use according to the invention, wherein the currently or repeatedly treatment for diabetes lasts for more than 2 months.
9. The insulin derivative for use according to the invention, wherein the currently or repeatedly treatment for diabetes lasts for more than 3 months.
10. The insulin derivative for use according to the invention, wherein the currently or repeatedly treatment for diabetes lasts for more than 1 year.
11. The insulin derivative for use according to the invention, for use in the treatment of diabetes, wherein the derivative is administered parenterally.
12. The insulin derivative for use according to the invention, for use in the treatment of diabetes, wherein the derivative is administered subcutaneously, intramusculary or intraveneously, and is preferably administered subcutaneously.
13. The insulin derivative for use according to the invention, for the treatment of diabetes, wherein said derivative is being administered to the same patient with a frequency in the range from every 2^{nd} day to every 11^{th} day.
14. A pharmaceutical formulation containing an insulin derivative for use according to the invention, for the treatment of diabetes, wherein said formulation is being administered to the same patient with a frequency in the range from every 2^{nd} day to every 11^{th} day.

### Example 1

**Effect of subcutaneously administered insulin analogue of the invention on blood glucose, C-peptide, and HbA1c versus NPH insulin and vehicle in Sprague Dawley rats**

Rats were allowed free access to food and water prior to the experiment. At time 0 min, 200 µL tongue blood was drawn. The rats (groups of 6 animals) were injected either with vehicle, NPH insulin (12 nmol/animal), and A14E, B16H, B25H, B29K(N^{ε}-eicosanedioyl-γGlu-[2-(2-{2-[2-(2-aminoethoxy)ethoxy]acetylamino}ethoxy)ethoxy]acetyl), desB30 human insulin (Compound 1) (50, 100, and 200 nmol/animal) s.c in the neck skin. At following time points, blood samples were drawn: 2, 4, 6, 8, 24, 32, 48, 72, and 96 hours. Blood samples were analysed for insulin exposure, C-peptide, plasma glucose, and HbA1c (HbA1c only 96 h time point).

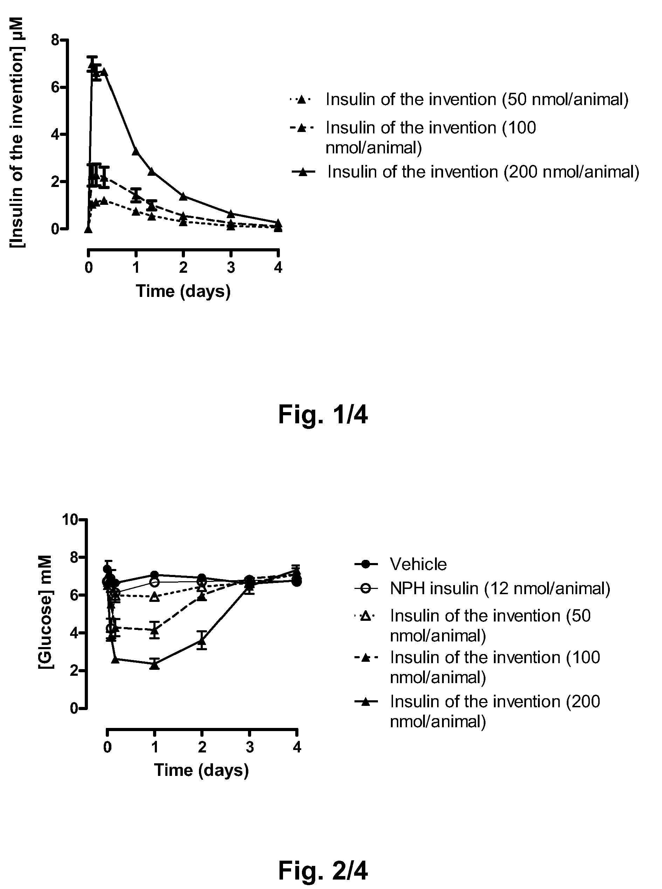

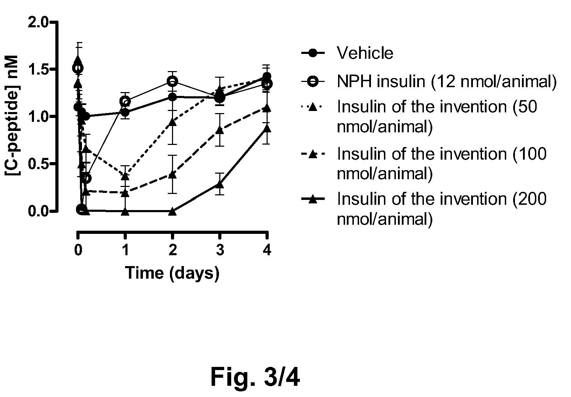

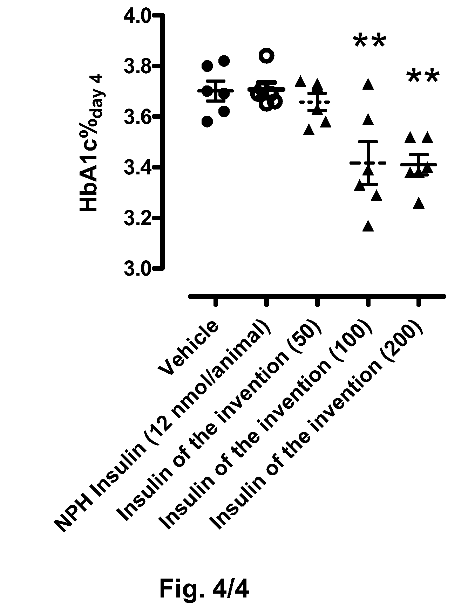

The results are presented in Figures 1-4:
Fig. 1 shows the plasma concentration levels following dosing of 3 different doses of Compound 1 (50, 100 and 200 nmol/animal) to rats within the time interval of 1-4 days.
Fig. 2 shows the blood glucose levels following dosing of NPH insulin (12 nmol/animal), of 3 different doses of Compound 1 (50, 100, and 200 nmol/animal) and of vehicle to rats within the time interval of 1-4 days.
Fig. 3 shows the C-peptide levels following dosing of NPH insulin (12 nmol/animal) and of 3 different doses of Compound 1 (50, 100, and 200 nmol/animal) to rats within the time interval of 1-4 days.
Fig. 4 shows the level of HbA1c 4 days after administration of NPH insulin (12 nmol/animal) and of 3 different doses of Compound 1 (50, 100, and 200 nmol/animal) to rats 4 days after administration.

The conclusion from these figures is that one single dosing of Compound 1 to normal non-diabetic rats is enough to:
- Lower blood glucose for 2-3 days
- Lower C-peptide for at least 4 days
- Significantly lower HbA1c in non-diabetic rats.

The importance of the data given for the compound of this invention is illustrated by comparison with the data for the NPH insulin which is, generally, considered a long-acting preparation.

### Example 2

Pharmacokinetic profiles after intravenously (i.v.) dosing were made in Beagle dogs (weighing approximately 12 kg) to evaluate the pharmacokinetic properties of insulin degludec (Insulin degludec is a once-daily basal insulin analogue with an ultra-long duration of action described in e.g. WO 2005/012347), and to evaluate the pharmacokinetic properties of A14E, B16H, B25H, B29K(N^{ε}-eicosanedioyl-yGlu-[2-(2-{2-[2-(2-aminoethoxy)ethoxy]-acetylamino}ethoxy)ethoxy]acetyl), desB30 human insulin (Compound 1). It was also desirable to be able to calculate the bioavailability after extravascular dosing. The animals were dosed i.v., 1.0 or 1.6 nmol/kg of insulin degludec or Compound 1, respectively, blood samples were collected and plasma analysed using sandwich immunoassay.

Pharmacokinetic profiles after subcutaneous (s.c.) profile were made in Beagle dogs (approximately 12 kg), to evaluate the pharmacokinetic properties after s.c. dosing. 4.1 or 17 nmol/kg of insulin degludec or Compound 1, respectively, were injected subcutaneously. Blood samples were drawn and the plasma concentration of insulin degludec and of Compound 1, respectively, were measured.

All studies were single dose studies. The calculations were performed using the software WinNonlin Professional 5.3 (Pharsight Inc., Mountain View, CA, USA). The data obtained are presented in Tables 1-2 below.

As can be deduced from the data obtained from MRT after s.c. dosing (7.7 hours versus 92 hours), it indicates that in dog the duration of action of Compound 1 is at least 7 times longer than the duration of action of insulin degludec, which in turn has been shown to be a true once daily insulin in humans.

**Table 1**

| Mean Dog PK data after i.v. dosing of insulin degludec and Compound 1, respectively | | | | | | | | |
|---|---|---|---|---|---|---|---|---|
| **Analogue** | **Dose nmol/kg** | | **AUC/D** **pM^{*}min/(nmol/kg)** | **AUC % extrap** | **Vz** **ml/kg** | **CL** **ml/kg/min** | **MRT** **h** | **T_{½}*** **h** |
| Insulin degludec | 1 | Mean | 3397 | 8 | 75 | 0.30 | 3.8 | 2.9 |
| (n=5) | | SD | 446 | 1 | 13 | 0.04 | 0.4 | 0.2 |
| Compound 1 | 1.6 | Mean | 67787 | 13 | 78 | 0.015 | 78 | 60 |
| (n=4) | | SD | 9506 | 0 | 12 | 0.002 | 2 | 1 |

| | | | | | | | | |
|---|---|---|---|---|---|---|---|---|
| * T_{½} given as harmonic mean±pseudoSD | | | | | | | | |

**Table 2**

| Mean Dog PK data after s.c. dosing of insulin degludec and Compound 1, respectively | | | | | | | |
|---|---|---|---|---|---|---|---|
| **Analogue** | **Dose** **nmol/kg** | | **Tₘₐₓ** *** H** | **Cₘₐₓ/D** **pM/(nmol/kg)** | **MRT** **H** | **T_{½}**** **h** | **F** **%** |
| Insulin degludec | 4.1 | Mean | 4 | 5495 | 7.7 | 6.1 | 80 |
| (n=5) | | SD | 1.1 | 1920 | 1.5 | 2.3 | 15 |
| Compound 1 | 17 | Mean | 24 | 6218 | 92 | 56 | 64 |
| (n=6) | | SD | 5 | 1250 | 2 | 2 | 13 |

| | | | | | | | |
|---|---|---|---|---|---|---|---|
| * Tₘₐₓ given as median±SD ** T_{½} given as harmonic mean±pseudoSD | | | | | | | |

| **Name** | **Explanation** |
|---|---|
| AUC | The area under the concentration-time curve from zero to infinity and the mean was calculated |
| | (WinNonlin name: AUCinf_pred) [Unit: pM*min] |
| AUC/D | The area under the concentration-time curve from zero to infinity divided with dose and the mean was calculated |
| | (WinNonlin name: AUCinf_pred/D) [Unit: pM*min/(pmol/kg)] |
| AUC %extrap | The percentage of the AUC from zero to infinity which was extrapolated from the last data point to infinity and the mean was calculated |
| | (WinNonlin name: AUC_%Extrap_pred) [Unit: %] |
| CL | The total body clearance and the mean was calculated |
| | (WinNonlin name: Cl_pred) [Unit: ml/(kg*min)] |
| Cmax | Plasma concentrations of the compound were measured and plotted against the time in a semi-logarithmic plot, and the time to reach peak or maximum concentration following a single dose is read directly from concentration-time profile. Cₘₐₓ is always associated with Tₘₐₓ. [Unit: pM] |
| Cₘₐₓ/Dose | Plasma concentrations of the compound were measured and plotted against the time in a semi-logarithmic plot, and the maximum (peak) plasma concentration after a single dose is read directly from concentration-time profile. Cₘₐₓ is always associated with Tₘₐₓ. [Unit: ml/(kg*min)] |
| D | Dose [Unit: nmol/kg] |
| F | The fraction sc bioavailable = ((AUCsc/Dsc)/(AUCiv/Div))*100 [Unit: %] |
| MRT | MRT = mean residence time extrapolated to infinity. The average time the number of molecules introduced into the body, resides in the body. MRT=AUMCINF_pred / AUCINF_pred and AUMC: Area under the first moment curve extrapolated to infinity was based on the last observed concentration and calculated as |
| | AUMClast+Clast/λz2+(Tlast+Clast)/ λz |
| | (WinNonlin name: MRTINF_pred) [Unit: Hrs] |
| T_{½} | The elimination phase can be described by the elimination half-life which expresses the time in which half of the compound disappears from plasma following administration and the harmonic mean was calculated |
| | (WinNonlin name: HL_lambda_z) [Unit: Hrs] |
| Tₘₐₓ | Plasma concentrations of the compound were measured and plotted against the time in a semi-logarithmic plot, and the time to reach peak or maximum concentration following a single dose is read directly from concentration-time profile. Tₘₐₓ is always associated with Cₘₐₓ and the median was given. [Unit: Minutes] |
| Vz | Volume of distribution based on the terminal phase = D/(lambda_z * AUC). The mean was calculated |
| | (WinNonlin name: Vz_pred) [Unit: Vz] |

### Example 3

Pharmacokinetic simulation was performed to show that A14E, B25H, B29K(N^{ε}-octadecandioyl-γGlu-2xOEG), desB30 human insulin (Compound 6) could be injected twice weekly to humans with similar flat steady state plasma concentration profile as insulin degludec injected every day.

The simulation was performed using the software WinNonlin Professional 5.3 (Pharsight Inc., Mountain View, CA, USA). A simple one compartment model with first order elimination and absorption rate was used to simulate insulin degludec dosing once daily (every 24 hour) and Compound 6, dosing once daily (every 24 hour) and in addition twice weekly (every 84 h). Average pharmacokinetic parameters from human studies were used for insulin degludec (absorption rate constant = 0.031 h⁻¹ and elimination rate constant = 0.25 h⁻¹ corresponding to absorption half-life of 22 hours and elimination half-life of 2.8 hours). For Compound 6, average parameters from human studies were used for the elimination rate constant, but as the absorption rate constant after subcutaneous dosing was not known for humans, the absorption rate constant for insulin degludec was used instead (elimination rate constant = 0.013 h⁻¹ corresponding to elimination half-life of 52 hours).

From the simulated steady state levels of both insulin analogues the maximum plasma concentration and the minimum plasma concentration achieved from plasma concentration vs time profiles was recorded. The fluctuation at steady state was then calculated as the maximum plasma concentration divided by the minimum plasma concentration.

The fluctuations were estimated to be:

| **Insulin analogue** | **Dosing interval** | **Fluctuation rate** |
|---|---|---|
| Insulin degludec | 24 hours | 1.5 |
| Compound 6 | 24 hours | 1.1 |
| Compound 6 | 84 hours | 1.5 |

Based on simulated results, Compound 6 given once daily would be expected to provide a more flat plasma concentration vs time profile than that insulin degludec as judged by the lowering in fluctuations rate. Also, Compound 6 injected twice weekly would be expected to give the same fluctuations in the plasma concentration profile as that of insulin degludec once daily based on similar fluctuation rates.

A14E, B25H, desB27, B29K(N^{ε}-(octadecandioyl-γGlu), desB30 human insulin (Compound 4) has been shown to have almost the same terminal half-life, and thus similar elimination rates, in humans as Compound 6. Also, A14E, B25H, desB27, B29K(N^{ε}-octadecanedioyl-γGlu-2xOEG), desB30 human insulin (Compound 5) is expected to show the same terminal half-life as that of Compound 6 in man (as the elimination half-lives are very similar to that of Compound 6 in dogs). Therefore a flat steady state plasma concentration profile with lower fluctuations than that of insulin degludec should be expected for these insulin analogues.

## Claims

1. An insulin derivative selected from the group consisting of
A14E, B16H, B25H, B29K(N^{ε}-eicosanedioyl-γGlu-[2-(2-{2-[2-(2-aminoethoxy)-ethoxy]acetylamino}ethoxy)ethoxy]acetyl), desB30 human insulin;
A14E, B16H, B25H, B29K(N^{ε}-hexadecandioyl-γGlu), desB30 human insulin;
A14E, B16H, B25H, B29K(N^{ε}-eicosanedioyl-γGlu), desB30 human insulin;
A14E, B25H, desB27, B29K(N^{ε}-octadecandioyl-γGlu), desB30 human insulin;
A14E, B25H, desB27, B29K(N^{ε}-octadecanedioyl-γGlu-OEG-OEG), desB30 human insulin; and
A14E, B25H, B29K(N^{ε}-octadecandioyl-γGlu-OEG-OEG), desB30 human insulin;
for use as a medicament for the treatment of diabetes, said compound being administered to the same patient with a frequency in the range from every 2^{nd} day to every 11^{th} day, during a period of time of at least 1 month, and within said period of time no administration of said derivative takes place to the same patient more frequently than every 2^{nd} day or less frequently than every 11^{th} day.

2. The insulin derivative for use according to claim 1, wherein said compound is administered during a period of time of at least 6 months.

3. The insulin derivative for use according to claim 1, wherein said compound is administered during a period of time of at least 1 year.

4. The insulin derivative for use according to claim 1, which is A14E, B16H, B25H, B29K(N^{ε}-eicosanedioyl-γGlu-[2-(2-{2-[2-(2-aminoethoxy)ethoxy]acetylamino}ethoxy)ethoxy]-acetyl), desB30 human insulin.

5. The insulin derivative for use according to claim 1, which is A14E, B16H, B25H, B29K(N^{ε}-hexadecandioyl-γGlu), desB30 human insulin.

6. The insulin derivative for use according to claim 1, which is A14E, B16H, B25H, B29K(N^{ε}-eicosanedioyl-γGlu), desB30 human insulin.

7. The insulin derivative for use according to claim 1, which is A14E, B25H, desB27, B29K(N^{ε}-octadecandioyl-γGlu), desB30 human insulin.

8. The insulin derivative for use according to claim 1, which is A14E, B25H, desB27, B29K(N^{ε}-octadecanedioyl-γGlu-OEG-OEG), desB30 human insulin.

9. The insulin derivative for use according to claim 1, which is A14E, B25H, B29K(N^{ε}-octadecandioyl-γGlu-OEG-OEG), desB30 human insulin.

10. A pharmaceutical formulation comprising an insulin derivative selected from the group consisting of
A14E, B16H, B25H, B29K(N^{ε}-eicosanedioyl-γGlu-[2-(2-{2-[2-(2-aminoethoxy) ethoxy]acetylamino}ethoxy)ethoxy]acetyl), desB30 human insulin;
A14E, B16H, B25H, B29K(N^{ε}-hexadecandioyl-γGlu), desB30 human insulin;
A14E, B16H, B25H, B29K(N^{ε}-eicosanedioyl-γGlu), desB30 human insulin;
A14E, B25H, desB27, B29K(N^{ε}-octadecandioyl-γGlu), desB30 human insulin;
A14E, B25H, desB27, B29K(N^{ε}-octadecanedioyl-γGlu-OEG-OEG), desB30 human insulin; and
A14E, B25H, B29K(N^{ε}-octadecandioyl-γGlu-OEG-OEG), desB30 human insulin;
for use in the treatment of diabetes, wherein said formulation is being administered to the same patient with a frequency in the range from every 2^{nd} day to every 11^{th} day.

## Patentansprüche

1. Insulinderivat, ausgewählt aus der Gruppe bestehend aus
A14E, B16H, B25H, B29K(N^{ε}Eicosandioyl-yGlu-[2-(2-{2-[2-(2-aminoethoxy)ethoxy]acetylamino}ethoxy)etho-xy]acetyl), desB30-Humaninsulin;
A14E, B16H, B25H, B29K(N^{ε}-Hexadecandioyl-γGlu), desB30-Humaninsulin,
A14E, B16H, B25H, B29K(N^{ε}-Eicosandioyl-γGlu), desB30-Humaninsulin,
A14E, B25H, desB27, B29K(N^{ε}-Octadecandioyl-γGlu), desB30-Humaninsulin,
A14E, B25H, desB27, B29K(N^{ε}-Octadecandioyl-γGlu-OEG-OEG), desB30-Humaninsulin und
A14E, B25H, B29K(N^{ε}-Octadecandioyl-γGlu-OEG-OEG), desB30-Humaninsulin,
zur Verwendung als Medikament zur Behandlung von Diabetes, wobei die Verbindung dem gleichen Patienten mit einer Häufigkeit im Bereich von jeden 2. Tag bis jeden 11. Tag über einen Zeitraum von mindestens 1 Monat verabreicht wird und wobei innerhalb des Zeitraums keine Verabreichung des Derivats an den gleichen Patienten mit einer Häufigkeit von mehr als jeden 2. Tag oder mit einer Häufigkeit von weniger als jeden 11. Tag stattfindet.

2. Insulinderivat zur Verwendung nach Anspruch 1, wobei die Verbindung über einen Zeitraum von mindestens 6 Monaten verabreicht wird.

3. Insulinderivat zur Verwendung nach Anspruch 1, wobei die Verbindung über einen Zeitraum von mindestens 1 Jahr verabreicht wird.

4. Insulinderivat zur Verwendung nach Anspruch 1, bei dem es sich um A14E, B16H, B25H, B29K(N^{ε}-Eicosandioyl-^{ε}Glu-[2-(2-{2-[2-(2aminoethoxy)ethoxy]acetylamino}ethoxy)ethoxy]acetyl), desB30-Humaninsulin handelt.

5. Insulinderivat zur Verwendung nach Anspruch 1, bei dem es sich um A14E, B16H, B25H, B29K(N^{ε}-Hexadecandioyl-γGlu), desB30-Humaninsulin handelt.

6. Insulinderivat zur Verwendung nach Anspruch 1, bei dem es sich um A14E, B16H, B25H, B29K(N^{ε}-Eicosandioyl-γGlu), desB30-Humaninsulin handelt.

7. Insulinderivat zur Verwendung nach Anspruch 1, bei dem es sich um A14E, B25H, desB27, B29K(N^{ε}-Octadecandioyl-γGlu), desB30-Humaninsulin handelt.

8. Insulinderivat zur Verwendung nach Anspruch 1, bei dem es sich um A14E, B25H, desB27, B29K(N^{ε}-Octadecandioyl-γGlu-OEG-OEG), desB30-Humaninsulin handelt.

9. Insulinderivat zur Verwendung nach Anspruch 1, bei dem es sich um A14E, B25H, B29K (N^{ε}-Octadecandioyl-γGlu-OEG-OEG), desB30-Humansinulin handelt.

10. Pharmazeutische Formulierung, umfassend ein Insulinderivat ausgewählt aus der Gruppe bestehend aus
A14E, B16H, B25H, B29K(N^{E}Eicosandioyl-yGlu-[2-(2-{2-[2-(2-aminoethoxy)ethoxy]acetylamino}ethoxy)ethoxy]acetyl), desB30-Humaninsulin;
A14E, B16H, B25H, B29K (N^{ε}-Hexadecandioyl-γGlu), desB30-Humaninsulin,
A14E, B16H, B25H, B29K(N^{ε}-Eicosandioyl-γGlu), desB30-Humaninsulin,
A14E, B25H, desB27, B29K(N^{ε}-Octadecandioyl-γGlu), desB30-Humaninsulin,
A14E, B25H, desB27, B29K(N`-Octadecandioyl-yGlu-OEG-OEG), desB30-Humaninsulin und
A14E, B25H, B29K(N^{ε}-Octadecandioyl-γGlu-OEG-OEG), desB30-Humaninsulin,
zur Verwendung bei der Behandlung von Diabetes, wobei die Formulierung dem gleichen Patienten mit einer Häufigkeit im Bereich von jeden 2. Tag bis jeden 11. Tag verabreicht wird.

## Revendications

1. Dérivé d'insuline choisi dans le groupe constitué de
A14E, B16H, B25H, B29K(N^{ε}-eicosanedioyl-γGlu-[2-(2-{2-[2-(2-aminoéthoxy)-éthoxy]acétylamino}éthoxy)éthoxy]acétyl), desB30-insuline humaine ;
A14E, B16H, B25H, B29K(N^{ε}-hexadécanedioyl-γGlu), desB30-insuline humaine ;
A14E, B16H, B25H, B29K(N^{ε}-eicosanedioyl-γGlu), desB30-insuline humaine ;
A14E, B25H, desB27, B29K(N^{ε}-octadécanedioyl-γGlu), desB30-insuline humaine ;
A14E, B25H, desB27, B29K(N^{ε}-octadécanedioyl-γGlu-OEG-OEG), desB30-insuline humaine ; et
A14E, B25H, B29K(N^{ε}-octadécanedioyl-γGlu-OEG-OEG), desB30-insuline humaine ;
pour utilisation en tant que médicament pour le traitement du diabète, ledit composé étant administré au même patient avec une fréquence dans la plage de tous les 2 jours à tous les 11 jours, pendant une période d'au moins 1 mois, et dans ladite période, aucune administration dudit dérivé n'est effectuée au même patient plus fréquemment que tous les 2 jours ou moins fréquemment que tous les 11 jours.

2. Dérivé d'insuline pour utilisation selon la revendication 1, ledit composé étant administré pendant une durée d'au moins 6 mois.

3. Dérivé d'insuline pour utilisation selon la revendication 1, ledit composé étant administré pendant une durée d'au moins 1 an.

4. Dérivé d'insuline pour utilisation selon la revendication 1, qui est A14E, B16H, B25H, B29K(N^{ε}-eicosanedioyl-γGlu-[2-(2-{2-[2-(2-aminoéthoxy)éthoxy]acétylamino}éthoxy)éthoxy]-acétyl), desB30-insuline humaine.

5. Dérivé d'insuline pour utilisation selon la revendication 1, qui est A14E, B16H, B25H, B29K(N^{ε}-hexadécanedioyl-γGlu), desB30-insuline humaine.

6. Dérivé d'insuline pour utilisation selon la revendication 1, qui est A14E, B16H, B25H, B29K(N^{ε}-eicosanedioyl-γGlu), desB30-insuline humaine.

7. Dérivé d'insuline pour utilisation selon la revendication 1, qui est A14E, B25H, desB27, B29K(N^{ε}-octadécanedioyl-γGlu), desB30-insuline humaine.

8. Dérivé d'insuline pour utilisation selon la revendication 1, qui est A14E, B25H, desB27, B29K (N^{ε}-octadécanedioyl-γGlu-OEG-OEG), desB30-insuline humaine.

9. Dérivé d'insuline pour utilisation selon la revendication 1, qui est A14E, B25H, B29K(N^{ε}-octadécanedioyl-γGlu-OEG-OEG), desB30-insuline humaine.

10. Formulation pharmaceutique comprenant un dérivé d'insuline choisi dans le groupe constitué de A14E, B16H, B25H, B29K(N^{ε}-eicosanedioyl-γGlu-[2-(2-{2-[2-(2-aminoéthoxy)-éthoxy]acétylamino}éthoxy)éthoxy]acétyl), desB30-insuline humaine ;
A14E, B16H, B25H, B29K(N^{ε}-hexadécanedioyl-γGlu), desB30-insuline humaine ;
A14E, B16H, B25H, B29K(N^{ε}-eicosanedioyl-γGlu), desB30-insuline humaine ;
A14E, B25H, desB27, B29K(N^{ε}-octadécanedioyl-γGlu), desB30-insuline humaine ;
A14E, B25H, desB27, B29K(N^{ε}-octadécanedioyl-γGlu-OEG-OEG), desB30-insuline humaine ; et
A14E, B25H, B29K(N^{ε}-octadécanedioyl-γGlu-OEG-OEG), desB30-insuline humaine ;
pour utilisation dans le traitement du diabète, ladite formulation étant administrée au même patient avec une fréquence dans la plage de tous les 2 jours à tous les 11 jours.
